# EUROPEAN PATENT APPLICATION

(11) **EP 3 607 985 A1**
(43) Date of publication of application: **12.02.2020**
(21) Application number: 18210873.8
(22) Date of filing: 06.12.2018
(51) Int. Cl.: A61M 15/00, A24F 47/00

(54) **A VAPORIZATION DEVICE**

(30) Priority: 09.08.2018 US 201816100150
(71) Applicant: GSW Creative Corporation, Santa Monica, CA 90401 (US)
(72) Inventor: Fornarelli, Thomas, Chicago, Illinois 60657 (US)
(74) Representative: Cooley (UK) LLP

(57) **Abstract**

Provided is a vaporization substance delivery device which has a battery (1), a coil (15), a vaporization chamber, a plurality of wicks (14, 16), and a tank (17) having a reservoir configured for housing a vaporization substance, where the vaporization substance delivery device vaporizes the vaporization substance; and is disposed in electronic communication with a microprocessor (11) that controls actuation of the vaporization substance delivery device based on a prescribed dosage regimen for the vaporization substance. The prescribed dosage regimen includes a preset dose delivery defined by a time duration of an actuation, and a time delay before initiation of a subsequent preset dose delivery. There is a timer in electronic communication with the microprocessor for tracking the time delay; a recording means in electronic communication with the microprocessor for recording the number of actuations and the time duration of each actuation; and a vibration control assembly. The microprocessor prevents actuation of the vaporization substance delivery device after an actuation has been completed until the time delay has passed. Also provided is a means for the microprocessor to verify that the preset dose delivery has been completed; a vibrator (9) in electronic communication with the microprocessor for actuating the vibrator; and a vibration control assembly.

## Description

### CROSS REFERENCE TO RELATED APPLICATIONS

This application is a continuation-in-part of application Ser. No. 15/158,572 filed May 18, 2016, and a continuation-in-part of application Ser. No. 15/181,336 filed June 13, 2016, the contents of each are incorporated by reference in their entirety, as if fully restated herein.

### TECHNICAL FIELD

The present disclosure relates to vaporization devices. More specifically, the present disclosure relates to a vaporization device assembly comprising programmed or preset with dose amount and time of dose information for a vaporization substance being delivered by said vaporization device assembly with a means for notifying a user of timing related to inhalation of vaporizable substance in a vaporization device, method of using the same, a charging case, a kit, and a vibration assembly.

### BACKGROUND

Personal vaporization devices which store substances to be vaporized include, for instance, inhalers for asthma treatment. Some personal vaporization devices provide feedback to a user. Some of the features for providing feedback to a user include a means of indicating by visual cue, audible cue, touch feedback, haptic, vibration, heat or other sensory signal, or prompt, that a device has reached a desired or predetermined vaporization temperature or for a predetermined time. This provides a user with information as to when a device is ready for use. For example, a simple LED may glow when the personal vaporization device is ready for inhalation, or provide different patterns, colors, or strengths programmed to give feedback related to consumption of the vaporized substance. Some of these features have also been adapted to the newer electronic cigarettes.

By using a custom vaporization element and microprocessor control, the vaporization device or vaporization device assembly limits the temperature of vaporization to ensure that overheating, or 'burning', of the need state oils does not occur. The temperature range of vaporization of cannabinoids and terpenes is far lower than that of the e-liquids that are used in standard electronic cigarette products, and to ensure consistent delivery of medicine this temperature must be properly controlled.

Personal vaporization devices being used for medical purposes can be equipped with additional feedback features which can give auditory and/or tactile warnings such as vibration, while the vibration can also be to disperse the active agents. Other gimmicks related to aerosol devices have also been created, such as providing a mechanism whereby a pack of e-cigarettes vibrates when within a certain preprogrammed distance from another pack of the same e-cigarettes, thus alerting the user as to a potential new supply.

In many vaporization products, manufacturers simply use off the shelf, standard commodity products for their components. However, many of these components do not have quality control standards or grading standards present in their manufacture, which can lead to situations where one batch of product can have a different grade of material used in the vaporization element and the second can contain something slightly different. This variance in the component makeup creates inconsistencies in the vaporization process, which removes the ability to deliver consistent dose delivery.

There remains, however, a need for connecting the personal vaporization device, and specifically an electronic cigarette, to the safe and precise delivery of medicinal vaporization substances or an active agent, such as a drug that is prescribed by a medical professional or available over the counter, using a programmed or preset dosing amount, and signaling at least one of a start and/or stop of an operation of the personal vaporization device via a simple haptic feedback, wherein the haptic feedback is controlled, and the device is lightweight, compact, portable, ergonomic, and durable to deliver the maximum amount of doses to the customer in the most user friendly manner possible.

### SUMMARY

The present disclosure relates to vaporization device which has a battery, a printed circuit board, and a microprocessor, a vaporization substance delivery means that produces a vapor, the microprocessor, in electronic communication with said vaporization substance delivery means, programmed or preset with dose amount and time of dose information for a vaporization substance being delivered by said vaporization substance delivery means. The start and stop of a certain operations of the vaporization device can be signaled by haptic feedback, such as vibration.

In one embodiment, said dose and time of dose information can set forth a number of actuations of said vaporization substance delivery means. The number of actuations can be at prescribed intervals and use a timer, in electronic communication with the printed circuit board and the microprocessor. The time between actuations of said vaporization substance delivery means can be tracked. The actuator can be connected to the vaporization substance delivery means and the microprocessor for actuating said vaporization substance delivery means, the actuator may also comprise a means for preventing actuation of said vaporization substance delivery means after the preset dose amount has been delivered by the vaporization substance delivery means.

In another embodiment, the means for the microprocessor to verify the preset dose amount has been delivered by the vaporization substance delivery means, and the start and stop of the programmed or preset dosage amount can be signed by a vibration comprising a vibrator in electronic communication with said printed circuit board and said microprocessor for actuating said vibrator. The vaporization device can comprise a means for controlling voracity of vibration of said vibrator, wherein the means comprises a vibration control assembly, a first spacer at the mouthpiece end and a second spacer at the vibration end, a printed circuit board, a vibrator and a microprocessor housed in the second spacer, a coil holder configured to accommodate a ceramic coil, a vaporization chamber, and a plurality of wicks, a coil for vaporizing substances, a tank having an reservoir, a first and a second end, wherein the reservoir is configured for housing vaporization substances, a reservoir plug which is accommodated in a first end of the tank; and a pressure sensor in pneumatic communication with the reservoir of the tank, wherein the battery at the vibration end is adjacent to and in electronic communication with the printed circuit board, the vibrator and the microprocessor which are contained in the second spacer, wherein the battery at the mouthpiece end is in electronic communication with the ceramic coil, and wherein the battery at the mouthpiece end is contiguous with the first spacer which separates the battery from the tank and the mouthpiece.

In another embodiment, the reservoir plug can be a molded component and comprise a ball shaped end. The reservoir plug can be utilized to perform at least two functions. One function is for the reservoir plug to seal the tank after the tank has been filled with the vaporization substance. A second function is for the reservoir plug to perform the function of a one-way valve. The reservoir plug can be fitted into the tank to create a vacuum inside the tank and covert the air pressure inside the tank into a vacuum for pumping the vaporization substance out of the tank at a constant rate.

In another embodiment, the vaporization device can comprise a recording means, coupled to said microprocessor, for recording a time and number of actuations of said vaporization substance delivery means, where the recording is actuated by an actuator.

In another embodiment, the vaporization device can comprise a reservoir for inhalant product used in electronic cigarettes and electronic pens which incorporates a bottom wall convex catch basin that, combined with a dual wick design and ensures that substantially all the inhalant product is directed to at least one of the dual wicks for maximized use of the inhalant product held therewithin. Therefore, regardless of how much or how little oil/liquid inhalant product is in the reservoir, it is always being directed toward at least one of the dual wicks so there is substantially no unusable space in the reservoir where inhalant product can collect and become waste.

In another embodiment, the wicks are not placed inside the tank. The wicks are in fluid communication with a screen. The vacuum pump created by the reservoir plug can provide a constant flow of vaporization substance out of the tank and onto the screen. The vaporization substance can temporarily adhere to the screen, and the screen can be in fluid communication with the plurality of wicks. The wicks draw the vaporization substance from the screen to the coil, and the vaporization substance is vaporized at a temperate that does not overheat the vaporization substance, causing a burnt taste.

In another embodiment, a charging case for a vaporization device comprises a lid, a cradle and a base, wherein the cradle is configured to secure a vaporization device in a position conducive for charging.

In another embodiment, a kit comprises a vaporization device, a charging case comprising a lid, a cradle and a base for charging the electronic cigarette; and a plurality of tanks filled with vaporizable substance.

In another embodiment, the vaporization device comprises a vibration control assembly for a vaporization device. The vibration control device comprises a spacer housing a printed circuit board, a vibrator, and a microprocessor for use in controlling the time at which the vibrator vibrates, the duration of the vibration, the strength of the vibration, and the pattern of the vibration, wherein the spacer is formed of a dampening material from the group consisting of plastic, rubber, silicon, and mixes thereof.

In another embodiment, a method for controlling vibration within a vaporization device is disclosed. The method comprises receiving, at a microprocessor, a signal of the start and/or stop at least one operation of the vaporization device. The method can comprise signaling the start and/or stop of at least one operation of the vaporization device using a sensor, such as a pressure or airflow sensor, in pneumatic communication with pneumatic communication with a reservoir of the vaporization device; determining, at the microprocessor, at least one of an initiation of inhalation and an end of a draw of the vaporization device, delivery of a programmed or present active agent dosage amount, engagement or disengagement of a battery, or other vaporization device operations, based on the signal from the pressure sensor, the microprocessor activating a vibrator based on the start or stop of at least one vaporization device operation, where the vaporization device has a means for controlling the veracity of the vibration.

In one embodiment, the means for controlling the veracity of the vibration can be a spacer that is formed of a dampening material from the group consisting of plastic, rubber, silicon, and mixes thereof, and wherein the spacer houses the printed circuit board, the vibrator and the microprocessor.

Known reservoirs typically incorporate a substantially centralized single siphon wick which allows inhalant product to be left in the tank which is unable to be wicked to the vaporizing area. Also, a single wicking point or wick increases the likelihood of failure if the wick should become clogged or degraded. Further, the prior art reservoirs all have a substantially, flat inside base or bottom wall with a wick about 7 cm higher than the base. What this means is that when the wick has vaporized the e-liquid inhalant product it can contact, there will typically always be a little product left at the bottom, below the wick which cannot be contacted by the wick and burned. When the wick becomes dried out and is burned, it produce an undesirable burned taste as it is not be able to burn all the remaining e-liquid inhalant product, and creates waste and means money lost for the buyer.

One proposed reservoir or tank of the present disclosure can comprise a convex shaped bottom wall. The material of the bottom is smooth so that the e-liquid inhalant product easily travels down to the outer bottom edge of the central convex shaped bottom wall to at least one of the dual wicks with which the bottom is in contact. The dual wicks delivers the e-liquid inhalant product to a vaporization chamber, and the convex shape of the bottom provides that substantially all of the e-liquid inhalant product will all be used, without any substantial waste and therefore substantially no money lost for the buyer. Moreover, the dual ended wick stays wet so that a burned taste is not produced.

Another proposed reservoir or tank of the present disclosure can comprise a screen in fluid communication with the tank, and wicks in fluid communication with the screen. This reservoir does not provide for the wicks being submerged in the vaporization substance inside the tank. The vaporization substance is pumped onto the screen at a constant rate using a reservoir plug as a valve that creates a vacuum inside the tank using air pressure. The vaporization substance temporarily adheres to the screen and the wicks pull the vaporization substance from the screen to the coil for vaporization of the vaporization substance..

One broad embodiment of the vaporization device comprises a vaporization substance delivery means that produces a vapor, a microprocessor connected to said vaporization substance delivery means that can be programmed or preset with dose amount and time of dose information for a vaporization substance being delivered by said vaporization substance delivery means, said dose and time of dose information setting forth a number of actuations of said vaporization substance delivery means to be provided at prescribed intervals, a timer connected to said microprocessor for tracking the time between actuations of said vaporization substance delivery means, a means, coupled to said microprocessor, for recording the a time and number of actuations of said vaporization substance delivery means, said recording being actuated by an actuator, said actuator connected to said vaporization substance delivery means and said microprocessor for actuating said vaporization substance delivery means, said actuator including means for preventing actuation of said vaporization substance delivery means after the preset dose amount has been delivered by the vaporization substance delivery means, means for the microprocessor verifying the preset dose amount has been delivered by the vaporization substance delivery means, a sensation device connected to said microprocessor for actuating said sensation device to signal the preset dose amount has been delivered by the vaporization substance delivery means, the timer to until the timer means a patient attempts to actuate the active agent delivery means, said verifying means comprising means for simultaneously sensing a patients mouth in close contact and around said active agent delivery means, a drop in pressure in said active agent delivery means and an actuation of said drug delivery means by said actuator.

In another broad embodiment, the vaporization device can comprise a battery with a mouthpiece end and an opposing vibration end, a first spacer at the mouthpiece end and a second spacer at the vibration end, a printed circuit board, a vibrator and a microprocessor housed in the second spacer, a coil holder configured to accommodate a ceramic coil, a vaporization cup, and a plurality of wicks, a screen, a ceramic coil for vaporizing substances accommodated by the coil holder, a tank having a reservoir, a first and a second end, wherein the reservoir is configured for housing vaporization substances, a reservoir plug which is accommodated in a first end of the tank, and a sensor, such as a pressure or airflow sensor, in pneumatic communication with the reservoir of the tank, wherein the battery at the vibration end is adjacent to and in electronic communication with the printed circuit board, the vibrator and the microprocessor which are contained in the second spacer, wherein the battery at the mouthpiece end is in electronic communication with the ceramic coil, and wherein the battery at the mouthpiece end is contiguous with the first spacer which separates the battery from the tank and the mouthpiece.

In another embodiment, the microprocessor can be programmed to activate the vibrator at the end of the draw and disconnects the battery from the coil thus preventing further vaporization of the substances.

In another embodiment, the microprocessor can be programmed to activate the vibrator based on preprogrammed pressure the time elapsed after initiation of inhalation.

In another embodiment, the charging case can comprise LED lights which exhibit a pattern illustrating one charging status from the group consisting of the vaporization device is charged, the case is charged, how charged the vaporization device is, how charged the case is, charging progress of the electronic cigarette, and charging progress of the case.

In another embodiment, a kit can comprise a vaporization device, a charging case comprising a lid, a cradle and a base for charging the electronic cigarette, and a plurality of tanks filled with at least vaporizable substance.

In another embodiment, the vaporizable substance can comprise an active agent.

In another embodiment, the active agent can be medicinal.

In another embodiment. a vibration control assembly for a vaporization device can comprising a spacer, a printed circuit board, a vibrator, and a microprocessor for use in controlling the time at which the vibrator vibrates, the duration of the vibration, the strength of the vibration, and the pattern of the vibration, wherein the spacer is formed of a dampening material from the group consisting of plastic, rubber, silicon, and mixes thereof, and wherein the spacer houses the printed circuit board, the vibrator and the microprocessor.

In another embodiment. a method for controlling vibration within a vaporization device can comprise receiving, at a microprocessor, a signal from a sensor, such as a pressure or airflow sensor, in pneumatic communication with pneumatic communication with a reservoir of the vaporization device, determining, at the microprocessor, at least one of an initiation of inhalation and an end of a draw of the vaporization device based on the signal from the pressure sensor, and activating a vibrator based on the at least one of the initiation of inhalation and the end of a draw of the vaporization device.

In another embodiment, the vibrator can be activated by electrically connecting a battery to a coil to enable vaporization.

In another embodiment, the vibrator can be deactivated by the stop of at least one operation of the vaporization device. The deactivating the vibrator can be disconnected from a battery and/or from a coil to prevent vaporization.

In another embodiment, the microprocessor can receive signal related to a duration of time, and the signal can be a second signal.

In another embodiment, the microprocessor can be programmed to determine an operation of at least one of an initiation of inhalation and an end of a draw of the vaporization device based on the signal from the pressure sensor and the second signal related to a duration of time.

In another embodiment, the vaporization device can have a reservoir for holding inhalant product for use in electronic cigarettes and electronic pens, comprising a smooth convex bottom wall catch basin within which substantially all inhalant product is contained, and providing dual wicks which are positioned diametrically opposite each other and are both engaged to a vaporization element for use in vaporizing the inhalant product.

In another embodiment, the vaporization device can comprise a catch basin and dual wicks provide for use of substantially all of the inhalant product without waste.

In another embodiment, the vaporization device can comprise diametric positioning of the wicks provides for at least one wick being in contact with the inhalant product regardless of the position of reservoir, if the wicks are in direct fluid communication or indirect fluid communication with the reservoir, and keeping the wicks from degrading or drying out and creating a burned taste.

In another embodiment, the vaporization device can comprise a smooth convex bottom wall catch basin made of a material that does not react with oxygen.

In another embodiment, the catch basin can be made of a material that does not react with water.

In another embodiment, the catch basin can be made of a silicon.

In another embodiment, the smooth convex bottom wall catch basin can be conical.

In another embodiment, the vaporization device can comprise a reservoir for holding inhalant product for use in electronic cigarettes and electronic pens with a smooth convex bottom wall catch basin within which substantially all inhalant product is contained, and wherein dual wicks are positioned diametrically opposite each other and are both engaged to a vaporization element for use in vaporizing the inhalant product.

In another embodiment, the catch basin and dual wicks can provide for use of substantially all of the inhalant product without waste.

In another embodiment, the reservoir can be diametrically positioned to provide for at least one wick being in contact with the inhalant product regardless of the position of reservoir and keeping the wicks from degrading or drying out and creating a burned taste.

In another embodiment, that battery can be a pulse modulated battery. The pulse modulated battery can be adapted to engage with a microprocessor through a feedback loop, flux circuit, or a combination thereof.

Other advantages and features of the disclosure will become apparent upon review of the detailed description and accompanying drawings.

### BRIEF DESCRIPTION OF THE DRAWINGS

FIG. 1 illustrates an exploded view of an embodiment of the vaporization device in accordance with the principles of the present disclosure;
FIG. 2 illustrates an embodiment of the charging case in accordance with the principles of the present disclosure;
FIG. 3 illustrates an embodiment of the charging case in accordance with the principles of the present disclosure;
FIG. 4 illustrates an embodiment of the vaporization device in accordance with the principles of the present disclosure;
FIG. 4A illustrates a top plan view of the vaporization device in accordance with the principles of the present disclosure;
FIG. 5 illustrates a partly assembled view of the vaporization device in accordance with the principles of the present disclosure, in accordance with the principles of the present disclosure;
FIG. 6 illustrates a cross section of the vaporization device in accordance with the principles of the present disclosure;
FIG. 8 shows a prior art single wick reservoir for an electronic cigarette and electronic pens, including a mouthpiece thereof;
FIG. 9 shows a longitudinal exterior view of one embodiment of the reservoir made in accordance with the teachings of the present disclosure; and
FIG. 10 shows a longitudinal cross sectional view of one embodiment of the reservoir of Figure 7 including the convex bottom wall catch basin with dual wicks of the present disclosure, and
FIG. 11 is a schematic drawing of the operational control members for disclosure.
FIG. 12 is a flow chart showing the operational control according to this disclosure.
FIG. 13 illustrates the device in assembled form without the cover and with the vibration assembly visible and another embodiment of the reservoir of FIG. 7 comprising the convex bottom wall catch basin with dual wicks and screen.

### DETAILED DESCRIPTION

The following detailed embodiments presented herein are for illustrative purposes. That is, these detailed embodiments are intended to be exemplary of the present disclosure for the purposes of providing and aiding a person skilled in the pertinent art to readily understand how to make and use the technology of the present disclosure.

Throughout this specification, unless the context requires otherwise, the word "comprise" or variations such as "comprises" or "comprising" or the term "includes" or variations, thereof, or the term "having" or variations thereof will be understood to imply the inclusion of a stated element or integer or group of elements or integers but not the exclusion of any other element or integer or group of elements or integers. In this regard, in construing the claim scope, an embodiment where one or more features is added to any of the claims is to be regarded as within the scope of the invention given that the essential features of the invention as claimed are included in such an embodiment.

Accordingly, the detailed discussion herein of one or more embodiments is not intended, nor is to be construed, to limit the metes and bounds of the patent protection afforded the present disclosure in which the scope of patent protection is intended to be defined by the claims and equivalents thereof. Therefore, embodiments not specifically addressed herein, such as adaptations, variations, modifications, and equivalent arrangements, should be and are considered to be implicitly disclosed by the illustrative embodiments and claims described herein and therefore fall within the scope of the present disclosure.

Additionally, it is important to note that each term used herein refers to that which a person skilled in the relevant art would understand such term to mean based on the contextual use of such term herein. To the extent that the meaning of a term used herein, as understood by the person skilled in the relevant art based on the contextual use of such term, differs in any way from any particular dictionary definition of such term, it is intended that the meaning of the term as understood by the person skilled in the relevant art should prevail.

Further, it should be understood that, although steps of various claimed methods may be shown and described as being in a sequence or temporal order, the steps of any such method are not limited to being carried out in any particular sequence or order, absent an indication otherwise. That is, the claimed method steps are considered capable of being carried out in any sequential combination or permutation order while still falling within the scope of the present disclosure.

One vaporization device assembly is disclosed in U.S. Patent Number 8,490,628, hereby incorporated by reference in its entirety. This disclosure provides for an electronic atomization cigarette that includes a shell and a mouthpiece. The external wall of the shell has an air inlet. It also includes an atomizer and a liquid-supply that are in contact with each other. The air inlet, atomizer, an aerosol passage, and a mouthpiece are sequentially interconnected.

Another vaporization device assembly is disclosed in U.S. Patent No. 8,881,737, hereby incorporated by reference in its entirety. This disclosure provides for electronic smoking article that provides for improved aerosol delivery. Particularly, the article comprises one or more microheaters. In various embodiments, the microheaters provide for improved control of vaporization of an aerosol precursor composition and provide for reduced power requirements to achieve consistent aerosolization. The present disclosure further relates to methods of forming an aerosol in a smoking article.

Another vaporization device assembly is disclosed in U.S. Patent No. 9,315,890, hereby incorporated by reference in its entirety. This disclosure provides for an electronic device for volatilizing organic matter is described. The electronic device includes a housing, a removable transparent tube, a heating element, a setting profile, a memory and a processor. The removable transparent tube has a chamber distal end within the housing. The heating element is proximate to the chamber distal end of the transparent tube. The chamber distal end holds vaporizable organic matter. The settings profile includes a first wattage setting and a first timer setting for the heating element and the heating element vaporizes the vaporizable material. The memory stores the settings profile. The processor is operatively coupled to the memory module and controls the heating element according to the settings profile.

Another vaporization device assembly and a method for using a vaporization device assembly are disclosed in U.S. Patent Application Number 20140190496, hereby incorporated by reference in its entirety. This disclosure provides for methods, devices, systems, and computer readable medium for delivering one or more compounds to a subject. Also described therein are methods, devices, systems, and computer readable medium for transitioning a smoker to an electronic nicotine delivery device and for smoking or nicotine cessation.

One type of dosing apparatus can be inhalation device with a dose-timer, an actuator, mechanism, and patient compliance monitoring means, such as the one disclosed in U.S. Patent No. 5,284,133, hereby incorporated by reference in its entirety.

One type of signal that can be used to signal the start or stop of the operation of the vaporization device is through haptic feedback produced by a sensation device. The sensation device can be a vibration assembly. The vaporization device has many operations that can be signaled, such as the start of vaporization, the stop of vaporization, the start of dose delivery, the stop of dose delivery, the engagement or disengagement of the battery, the engagement or disengagement of the microprocessor, the engagement or disengagement of the actuator, the start or stop of an electric process, and other vaporization device operations. One disclosure of a vaporization device that is adapted to use haptic feedback is U.S. Patent Application 20150020825, hereby incorporated by reference in its entirety. The disclosure provides for an electronic smoking article a smoking article that can comprise a housing that includes a haptic feedback component, such as a vibration transducer. The smoking article can be formed of a control body and/or a cartridge, and the haptic feedback component may be present in any one or both of the control body and the cartridge. The haptic feedback component is adapted to generate a waveform that defines a status of the electronic smoking article. The disclosure also provides a method for providing haptic feedback in an electronic smoking article.

One type of pulse modulated battery is disclosed in U.S. Patent No. 5,296,797. The disclosure provides for a pulse width modulated battery charging system that applies constant current at a set voltage acceptable to the battery under charge and applied by a 90% duty cycle pulse until approximately two thirds of a full battery condition is reached, followed by gradually diminishing pulse width to 10% of said current and voltage as full charge is approached, and terminated when full charge is reached, and characterized by sensing battery condition during dwell between pulse charges and increasing the dwell for battery recovery from the affects of previous pulse charges.

Another type of pulse modulated battery is disclosed in U.S. Patent No. 7,683,574, incorporated by reference in its entirety. The disclosure provides for a rapid charging circuit for a lithium ion battery. The battery charger in accordance with the present invention compensates for the voltage drops across the various resistance elements in the battery circuit by setting the charging voltage to a level to compensate for the initial resistance of the series resistances in the circuit and an additional resistance selected to take into account the anticipated increase in resistance of the various circuit elements over time. The battery charger in accordance with the present invention periodically monitors the open-circuit voltage of the battery cell and reduces the charging voltage to when the battery cell voltage reaches the optimal value. Thus, during a constant current charging mode, the battery cell is driven at a relatively optimal charging current to reduce the charging time. As such, the system is able to optimize the charging current supplied to a battery cell during a constant current mode of operation while compensating for circuit elements whose resistance may vary over time due to temperature or other factors, such as corrosion, while at the same time avoiding exceeding the maximum recommended voltage for the battery cell.

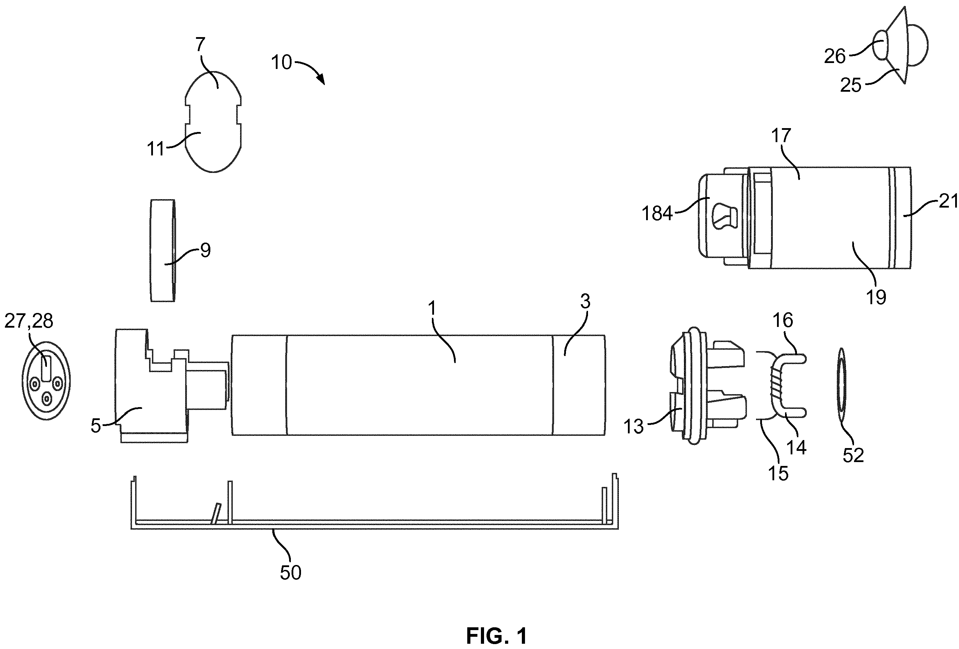

FIG. 1 illustrates an embodiment of the vaporization device assembly 10 of the present disclosure in an exploded and unassembled view. In the embodiment is provided the battery 1 which supplies electricity by which the microprocessor 11 and the printed circuit board 7 ("PCB") activate the method of vaporization and controlling the same of the assembly.

In one embodiment, the battery 1 must maintain a proper voltage range throughout the life of the assembly 10. The life of the assembly 10 lasts from about 60-120 doses, 70-130 doses, 80-120 doses, 150 to 250 doses, 175 to 225 doses, 190 to 215 doses, or 195 to 205 doses. If the voltage of the battery 1 drops below its optimum power range during the life of the dose pen, the vaporization profile can change, reducing the amount of vapor delivered in each activation of assembly 10. In order to achieve a consistent active agent release/dose amounts throughout the life of the assembly, a battery that is capable of providing the mAh needed to deliver the size of the assembly 10. The assembly 10 may be made to provide, for instance, 90 doses. The assembly 10 may be made to provide, for instance 200 doses. The battery 1 must be the right size to provide the number of doses the assembly 10 is made to provide. As an example, for a 200 dose assembly 10, the batter 1 must provide about 220 mAh to 260 mAh and delivering about 220 mAh 260 mAh each time it is engaged, is preferred. For the 90 dose assembly 10, the mAh provided by the battery 1 can be less than the mAh provided by the batter 1 for the 200 dose assembly 10.

In one embodiment, the battery 1 can be a 220mAh lithium-ion battery that is sized to specifically deliver the full amount of programmed or preset doses of the vaporization substance.

In one embodiment, battery 1 provides power to the vaporizing element 20, the microprocessor 11, the vibration assembly 200, and at least one of a plurality of sensors 27. Sensors can include capacitive sensing components that are incorporated into the device in a variety of manners to allow for diverse types of "power-up" and/or "power-down" for one or more components of the device. Capacitive sensing can include the use of any sensor incorporating technology based on capacitive coupling including, but not limited to, sensors that detect and/or measure proximity, position or displacement, humidity, fluid level, airflow, pressure, temperature, or acceleration. Capacitive sensing can arise from electronic components providing for surface capacitance, projected capacitance, mutual capacitance, or self-capacitance. Capacitive sensors generally can detect anything that is conductive or has a dielectric different than that of stagnant air. Capacitive sensors, for example, can replace mechanical buttons (i.e., the push-button referenced above) with capacitive alternatives. Thus, one specific application of capacitive sensing according to the invention is a touch capacitive sensor. For example, a touch pad can be present on the smoking article that allows the user to input a variety of commands. Most basically, the touch pad can provide for powering the heating element much in the same manner as a push button, as already described above. In other embodiments, capacitive sensing can be applied near the mouth end of the smoking article such that the pressure of the lips on the smoking article to draw on the article can signal the device to provide power to the heating element. In addition to touch capacitance sensors, motion capacitance sensors, liquid capacitance sensors, and accelerometers can be utilized according to the invention to elicit a variety of response from the smoking article.

Further, photoelectric sensors also can be incorporated into the inventive smoking article. A self-contained photoelectric sensor contains the optics, along with the electronics. It requires only a power source. The sensor performs its own modulation, demodulation, amplification, and output switching. Some self-contained sensors provide such options as built-in control timers or counters. Because of technological progress, self-contained photoelectric sensors have become increasingly smaller. Remote photoelectric sensors used for remote sensing contain only the optical components of a sensor. The circuitry for power input, amplification, and output switching are located elsewhere, typically in a control panel. This allows the sensor, itself, to be very small. Also, the controls for the sensor are more accessible, since they may be bigger.

When space is restricted or the environment too hostile even for remote sensors, fiber optics may be used. Fiber optics are passive mechanical sensing components. They may be used with either remote or self-contained sensors. They have no electrical circuitry and no moving parts, and can safely pipe light into and out of hostile environments.

Photoelectrical sensors, such as an optical sensor, can expressly signal for power flow to the heating element so as to heat the vaporization substance and form a vapor or aerosol for inhalation by a user. Sensors also can provide further functions. For example, a "wake-up" sensor can be included. Other sensing methods providing similar function likewise can be utilized according to the invention.

In one embodiment, the microprocessor 11 is adapted printed circuit board ("PCB") 7. The microprocessor 11 can control the vaporization time, control the power supplied to the vaporization element 20 by the battery 1, and the time and count of the vibration device 10.

In one embodiment, the PCB 7 may include, for example, but is not limited to, a processing unit, a memory unit, a plurality of timers, and other suitable electrical components. Electronic components of the vaporization device assembly 10 are fixed to the PCB 7, which mechanically supports and electrically connects components of the assembly 7 using tracks, pads, and other features etched from conductive sheets laminated onto a non-conductive substrate. In some embodiments, the electronics of the PCB 7 is composed of a synthetic material that is thin and flexible. A thin and flexible PCB 7 allows the same to conform to the shape of the electronic cigarette. A PCB 7 is composed of materials such as, but not limited to, polyimide, polyethylene naphthalate, poletherimide, fluoropolymers, transparent conductive polyester, and other suitable materials for flexible electronics.

The battery 1 of the assembly 10 can be rechargeable, can be recharged/charged via induction charging, and/or can be charged by a wall electrical outlet, and/or by accommodating a USB to a computer to recharge/charge. In some embodiments, battery 1 can be a pulse modulated battery, a lithium battery, a lithium-ion battery, a nickel-cadmium ("NiCd") battery, a nickel-metal hydride ("NiMH") battery, or another suitable battery type. The battery 1 of the assembly 10 will be of suitable shape and length to essentially provide the look and feel of an electronic cigarette.

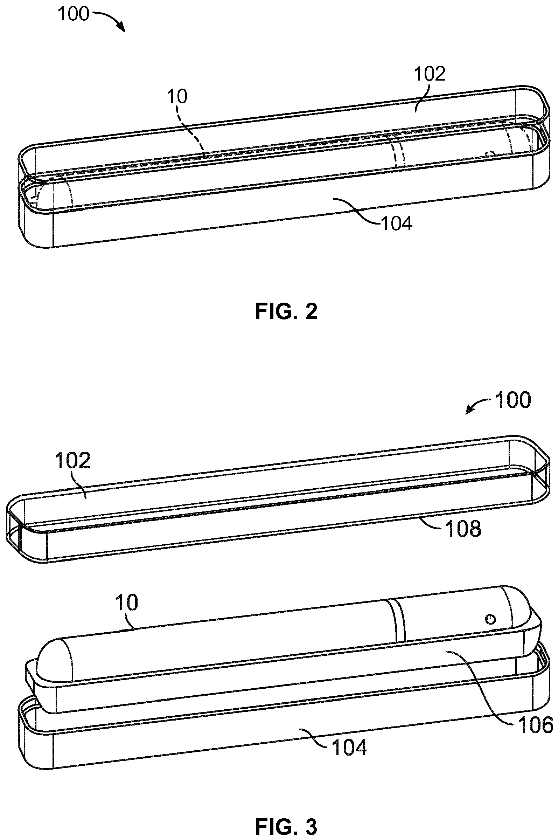

FIG. 2 illustrates an embodiment of the vaporization device 10 of the present disclosure in an embodiment of a charging case 100 of the present disclosure. The charging case 100 has a lid 102 and a base 104 which is configured to snuggly accommodate the vaporization device 10 which is visible through the transparent lid 102. It is not necessary that the lid 102 be transparent. In another embodiment the lid 102 is not transparent, is any color, and/or is translucent or opaque, in whole or in part. FIG. 3 illustrates a semi-exploded view of the case 100 of FIG. 2 wherein the lid 102 is partially hidden at the top right corner. In this view it is shown that there is a cradle in which the vaporization device 10 is cradled in cradle 106 to provide a good fit in the case 100. The lip 102 has a lip edge 108 which is compatible with the base edge 110 so as to engage and retain each other to close and hold the case 100 in the closed position.

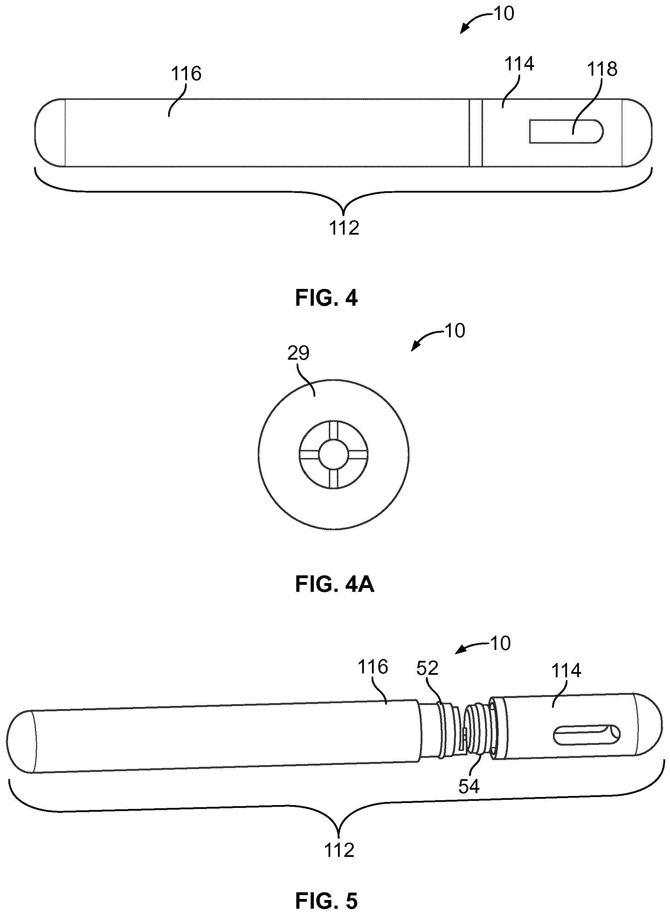

In FIGS. 2 and 3, and in FIG. 4 is illustrated the vaporization device 10 encased in a two-part cover 112. The cover 112 is in two parts and is provided with a shorter mouthpiece end cover 114 and a longer vibration end cover 116. The shorter end 114 is configured to accommodate the tank 17 while the longer end 116 is configured to accommodate the battery 1, first and second spacer 3,5, the vibrator 9, the plurality of pressure sensors 27, the PCB 7 and the microprocessor 11. The case 112 is preferably made from medical grade plastic. The shorter end 114 in this embodiment is also provided with a window 118 for the convenience of the user to be able to identify whether there is a tank 17 in the vaporization device 10 at all, and if so, the level of vaporization substance therein. FIG 4A illustrates the top plan view of the assembly 10. The top and bottom ends of the assembly 10 are mirror images. Therefore, the tip 29, can be of any shape, and in this embodiment is rounded at both ends and can also be provided with a vortex movement such as that of a windmill, as long as an air passage is provided for drawing air through the device to the mouthpiece end. Moreover, in this embodiment, in the center of the tip 29 is a screened air passage for the vaporization process. This screen can prevent particulate matter from entering the device and potentially damaging or interfering with the functioning thereof.

Turning back to FIG. 3, the cradle 106 further serves to stabilize the vaporization device assembly 10 in the case 100 in the event that the battery is rechargeable. In such an embodiment the placement of the vaporization device assembly 10 inside the case 100 allows a charged case 100 to charge a vaporization device assembly 10. In an embodiment wherein the case 100 is rechargeable, the case 100 can be recharged/charged via induction charging, and/or can be charged by a wall electrical outlet, and/or by accommodating a USB to a computer to recharge/charge. Preferably the base 104 of the case is provided with a USB outlet and/or a cord outlet for direct charging from the wall. The case 100 can also be provided with LED lights such that a pattern for charging is detected on the case 100. For example, but not limited to, a pattern whereby lights flash in a pattern when the vaporization device assembly 10 is being charged, and a different pattern when finished and only the case 100 is being charged.

FIG. 4A illustrates a tip 29 of the vaporization device 10.

FIG. 5 illustrates the cover 112 being assembled over the vaporization device assembly 10 the two parts 114, 116 of the cover 112 can be joined by any means understood by a person in the art to securely and smoothly fit the two pieces together. In the embodiment in FIG. 5 is shown an O-ring 52 for a lip and groove snap fit for retaining the two parts 114, 116 in a closed position. Also shown is an O-ring 54 on the internally located coil holder 13 for stably affixing the coil 15 in the cover 112.

Turning back to FIG. 1, it further illustrates on the mouthpiece end of the battery 1 a first spacer 3. This first spacer 3 is made of dampening material that does, such as, for instance, a rubber or a foam rubber material, or equivalent thereof. When the vaporization device assembly 10 is assembled, the first spacer 3 is located adjacent to coil holder containing a plurality of wicks 14, 16. The wick holder 13 is kept in place in the tank 17 by the use of an O-ring 52, see for example FIG. 5 and FIG. 1. This prevents the movement of the wicks 14, 16 and coil holder 13 in the vaporization device assembly 10 and cover 112, thus reducing a chance of damaging the electrical components or leaking any of the vaporization substance from the tank 17. A cap 16 is also provided in the tank to further protect the plurality of wicks 14, 16. The wicks 14, 16 can be any material understood to be effective for vaporizing substances, for instance, but not limited to, ceramic.

In general e-liquid tanks are filled with an oil based substance which is vaporized and provides at least one of flavors and active agents, such as medicinals prescribed by a doctor or available over the counter. The tanks are provided with a small delivery aperture, which makes it difficult to accurately fill the tank for filling prescriptions and medicaments, but also makes a mess which could in turn ruin the electronics if exposed to the oils. Imprecise filling of the tanks results in loss of vaporizable substance, potentially the electronics of the device, and therefore, can be costly. Moreover, medical prescriptions require consistent and precise dosages, and at the same time require medical grace preparation, vaporization substance and materials, which is not possible with leaky and imperfect filling. This problem is solved by at the first end of the tank, providing a reservoir plug 25.

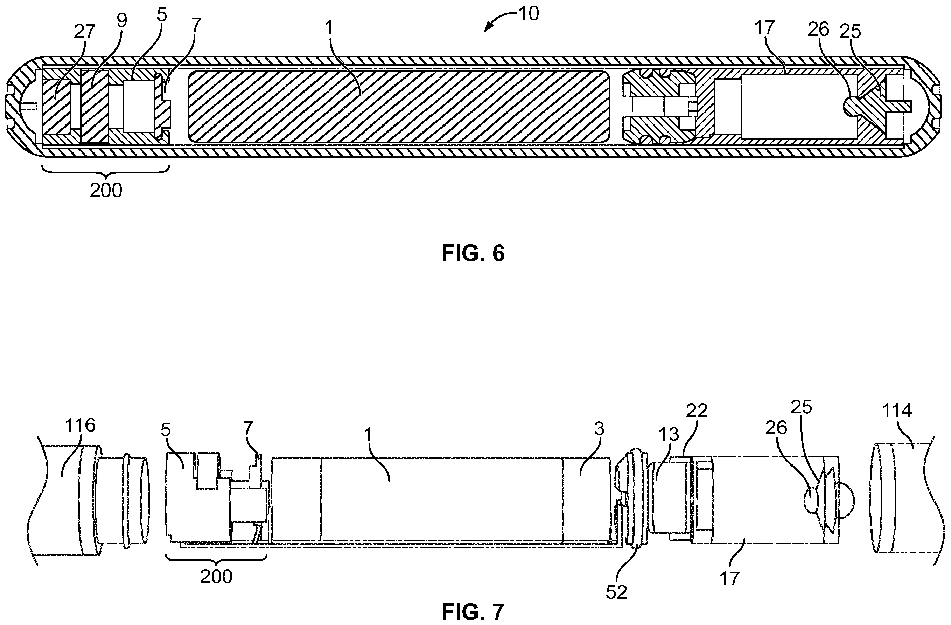

FIG. 6 illustrates the vibration assembly 200 for use in a vaporization device, as described herein. The vibration assembly 200 has a vibrator 9, a PCB 7, a microprocessor 11, all embedded in a spacer 5 which snuggly fits each component for immovable and compact localization within the vaporization device 10.

In FIG. 6 can be seen the reservoir 25 inserted into the first end of the tank such that the locking ball shape 26 catches the top of the tank preventing dislodgement. Moreover, the cone shape of the reservoir 25 provides a funnel design that offers a broader surface by which to guide a filling needle. This shape of the reservoir 25 allows for more accurate filling with much less vaporizable substance lost outside the reservoir 19 of the tank 17 thus sparing the electronic components of the vaporization device assembly 10 while saving money. Moreover, the shape of the reservoir 25 prevents the leaking of the vaporizable substance when already filled into the reservoir 19 because the molded reservoir 25 having a ball shaped end 26 secures the same.

The vibration end of the battery 1 in FIG. 6 abuts the second spacer 5. The second spacer 5 is configured to specifically accommodate the shape of a pressure sensor 28, the PCB 7 and microprocessor 11, and the vibrator 9. The second spacer 5 and components housed thereby is referred to as a vibration assembly 200. The second spacer 5 is made of a plastic or dampening material. For example, in one embodiment it is made of a material that does not react with oxygen, such as silicone. The material of the second spacer 5 which surrounds the vibrator 9 and in which it sits, dampens the vibration. In addition, the distance from the mouthpiece of the user provides a mechanism for dampening the vibration of the vibrator 9, and also the first spacer 3, contiguous with the mouthpiece end of the battery 1, dampens the strength of the vibration on the mouth of the user. Even the outside two-part cover 112 of the assembly 10 has a dampening effect. This is important because although the vibrator 9 is pre-programmed to have a low voltage of for example, but not limited to, 0.01 volts, lips are sensitive and a user could still receive a surprise given a vibration stronger than expected at the mouth. By providing a location, surrounding materials, and a configuration conducive to dampening the effect of the vibration so that it is felt last at the lips, the assembly 10 provides a new and more comfortable way of giving feedback to the user.

The vibrator 9 feature of the assembly 10 could be activated upon a user initially inhaling whereby the vibrator 9 is tied to a pressure sensor 28 detecting inhalation and therefore vibrating at the beginning of the draw. By "draw" it is meant to inhale through the assembly at the mouthpiece end. The pressure sensor 28 may be, for example, but not limited to, a pressure responsive transducer, such as a piezo resistive transducer, having an electrical resistance that varies depending upon the air pressure to which it is subjected from the reservoir 19. Other forms of electronic transducers capable of producing an analog signal in response to air pressure can be utilized for the pressure sensor 28. The pressor sensor 28 is pneumatically connected to the reservoir 19 by at least one airway/duct. FIG. 1 provides for at least one airway/duct 50 along with a plurality of electrical communication wires connecting the various components of the vibration assembly to the reservoir 19 and/or tank 17.

The vibrator 9 feature could also be activated at the beginning and remain active as long as inhalation pressure is detected by at least one of a plurality of sensors 27. The vibrator 9 feature could also be activated at the end of the draw. This last embodiment could be useful to those users who are using the assembly 10 for taking doses of prescribed medicaments whereby as the user inhales, a pressure change is detected by the pressure sensor 28 and relayed to the vibrator 9 through the microprocessor 11 which at a preprogrammed time after the initially detected change in pressure in the tank, for instance 1-5 seconds, 2-5 seconds, 3-5 seconds, 4-5, seconds, 5 seconds, 1-4 seconds 2-5 seconds, 3-5 seconds, 2-4 seconds, 3-4 seconds, 1 second, 2 seconds, 3 seconds, or 4 seconds. Most preferably, the activation time is about 3 seconds.

Creating and programming a timed activation period for each active agent release/dose ensures that a vaporization element 20 is engaged for a specific length of time for each draw. The limited time of activation, which can be specified to meet the needs of the majority of the patient population, controlled by microprocessor 11. Microprocessor 11 controls the time of vaporization, the temperature of vaporization, and engaged the vibrator 9 to produce haptic feedback that can signal the end of the active agent release/dose.

After the preprogrammed time concludes, the vibrator 9 is activated to alert the user to the end of the dose. In this way, a more accurate dose is delivered and no vaporizable substance is wasted (and battery charge). Moreover, a delay between the beginning of the change in pressure and the end of the dose alert provides time for the vaporization of the substance.

The assembly 10 comprises a screen 22 to ensure a consistent flow of vaporization substance to the vaporization element 20. By separating the reservoir 19 and/or tank 17 from the vaporization element 20 and controlling the flow of the vaporization substance to the vaporization element 20, the quantity of vaporization substance vaporized during each active agent release/dose remains constant.

The screen 22 can be a capillary screen that uses adhesion, such as surface tension, to adhere the vaporization substance to the screen before it is delivered to the vaporization element 20. The vaporization substance travels from the resivorl9 or tank 17 to the screen 22, and the wicks 14, 16 wick the vaporization substance off the screen 22.... that are held in place by wick holder 13. The screen 22 may be made from a material that has anti-static properties, such as ceramic. The vaporization substance can be pumped using a vacuum pump created by the reservoir plug 25 functioning as a one-way valve and using air pressure to pump the vaporization substance onto the screen 22 at a nearly constant rate to keep the screen 22 covered in enough vaporization substance so that it does not dry out.

To further illustrate how the assembly 10 could be even more useful in dosage precision, the activation of the vibrator could at the same time open the circuit of the battery to the coil thus alleviating the vaporization and ending the dose automatically. In this way, a weaker inhaler, for instance, will remain confident that the dose was administered because the change in pressure indicates that the dose amount of vaporizable substance was vaporized (i.e. removed from the chamber), triggering the change in pressure events. The amount of time between the beginning of the vibration and the change in pressure could be different depending on the prescribed medicinal, due to differences in vaporization time and temperature. Moreover, the amount of time that the vibrator vibrates could also be different depending not only on that, but also on the subjective inhalation strength of the user/patient. For instance, a patient with a strong inhalation may need a shorter vibration time and vice versa for a weaker inhaler.

Once the substance is vaporized, the dose is complete in the event of a medicinal, or the event is over in the event of a recreational user. Even tobacco substances can be used with this device. The tanks 17 can be replaceable. It is also contemplated that the tanks 17 of the assembly are disposable. Moreover, in yet another embodiment, the entire assembly is disposable and cannot be reused.

In addition, light-emitting diode ("LED") lights can be provided at the vibration end of the assembly 10 whereby the lights are on when the draw is initiated, and off when not, or any other pattern including fading during the duration of the draw. LED lights could also be provided to emphasize the vaporizable substance in the tank 17 in order to easily view the contents therein. The LED lights could be different colors, or change colors during operation of the assembly 10. In some embodiments, the light flashes. In another embodiment, the color may go across the length or width or diagonal of the assembly 10.

It is also possible that the tactile vibration effect be coupled with other means known in the art to alert a user as to dosage and usage.

The vibrator 9 outputs vibrations to notify the user (e.g., causing the container 10 to move). The vibrator 9 uses, for example, a motor coupled to a plurality of weights, or an eccentric cam system. In some embodiments, a rotational motor causes the plurality of weights to rotate. Each of the plurality of weights may have a different mass, causing the motor and the plurality of weights to rotate unevenly; the uneven rotation leads to vibration. In other embodiments, a linear motor causes the plurality of weights to move.

The vibrator 9 may be, for example, but is not limited to, 6 to 8 millimeters in diameter and/or 3-4 millimeters in length. The vibrator can be sized so that it fits within the second spacer 5.

The vibrator 9 is capable of vibrating a various frequencies depending on the electricity supplied by the battery 1. In some embodiments, the vibrator 9 will rotate/vibrate at 10 Hertz (in other words, 600 rpm) when supplied less than 0.2 Volts from the battery 1. In other embodiments, the vibrator 9 will rotate/vibrate at 100 Hertz (in other words, 6,000 rpm) when supplied more than 1 Volt from the battery 1. The vibration frequency can be any frequency or rpm that is desired.

In one embodiment, the microprocessor 11 receives a signal from at least one of the plurality of sensors 27 and/or pressure sensor 28 based on the air pressure within the reservoir 19 of the tank 17. The signal is indicative of an initiation of an inhalation and/or the end of a draw. The microprocessor 11 determines, at the microprocessor, at least one of an initiation of inhalation and an end of a draw of the vaporization device based on the signal from the pressure sensor. In some embodiments, the microprocessor 11 also receives a second signal related to a duration of time. The duration of time is, for example, but not limited to, how long a user/patient is inhaling/drawing the substance from the vaporization device, engagement and disengagement of the battery 1, the start of vaporization, the stop of vaporization, the start of dose delivery, the stop of dose delivery, the engagement or disengagement of the microprocessor 11, the engagement or disengagement of the actuator, the start or stop of an electric process, the start and stop of the timer 122, and other vaporization device operations. Next, the microprocessor 11 activates the vibrator 9 based on the at least one of the operations listed above.

The microprocessor 11 activating the vibrator 9 may consist of, for example, electrically connecting the battery 1 to a coil 15 to enable vaporization. The microprocessor 11 may deactivate the vibrator 9 based on the signal from the pressure sensor 27 and/or the duration of time. Deactivating the vibrator 9 consists of, for example disconnecting the battery 1 from the coil to prevent vaporization.

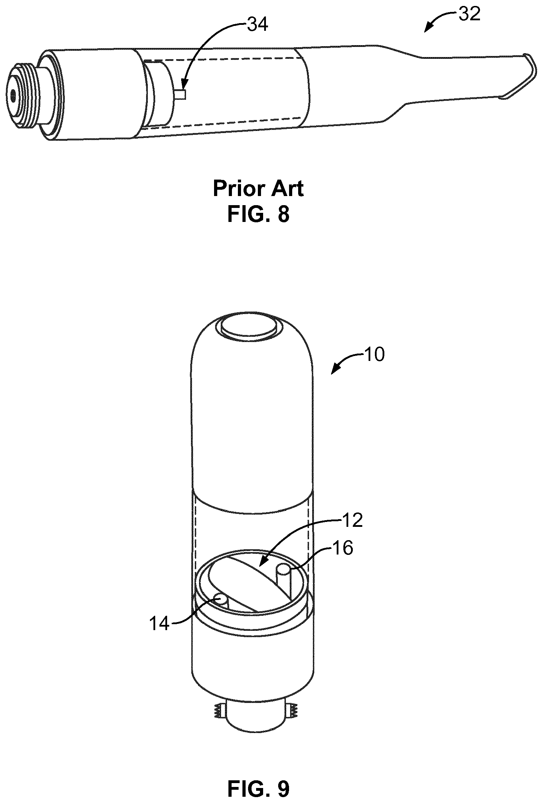

FIG. 8 illustrates a standard prior art cartridge or reservoir. In a standard prior art cartridge or reservoir, the wick is usually a one-piece siphon wick, which sticks up through the middle of the tank or reservoir. This one-piece siphon wick creates two primary functionality issues. First, there is approximately 5 millimeters of space where inhalant product can collect underneath the wick which leads to a significant portion of the product remaining unused and unusable in the reservoir. This is especially true for thicker oil vaporization substances, which tend to collect along the bottom of the reservoir near a spacer (not shown) and cannot be moved to the wick unless the reservoir is heated and tilted, and even then there is no way to get all of the collected oil product to the wick. Secondly, because there is only a single pathway from the top of the wick through the silicon component and into the vaporization coil, any overload of the wick's capacity or degradation of the wick material due to plant matter and/or essential fatty acids in the plant oil creates a product failure either through leaking or through loss of functionality of the wicking process, which leads to no oil being delivered to the vaporization element. This creates the very common "burnt" or "metallic" taste which is produced by such single wick reservoirs.

In FIG. 9, one embodiment of the present disclosure is illustrated. Tank 17 including the wicks 14, 16, as well as other device 10 components, are shown from its exterior, and the issues noted in the prior art have been addressed through the design of a molded, convex or conical silicon bottom wall catch basin 12 and a dual wick 14, 16 design. The convex or conical smooth bottom wall 12 ensures that all oil inhalant product (not shown) is funneled to at least one of the two wicks 14, 16 in the reservoir 19 or tank 17, regardless of how much or how little oil inhalant product (not shown) is in the tank 17 and reservoir 19. In one embodiment, the catch basin 12 is made of a material that does not react with oxygen, such as silicon.

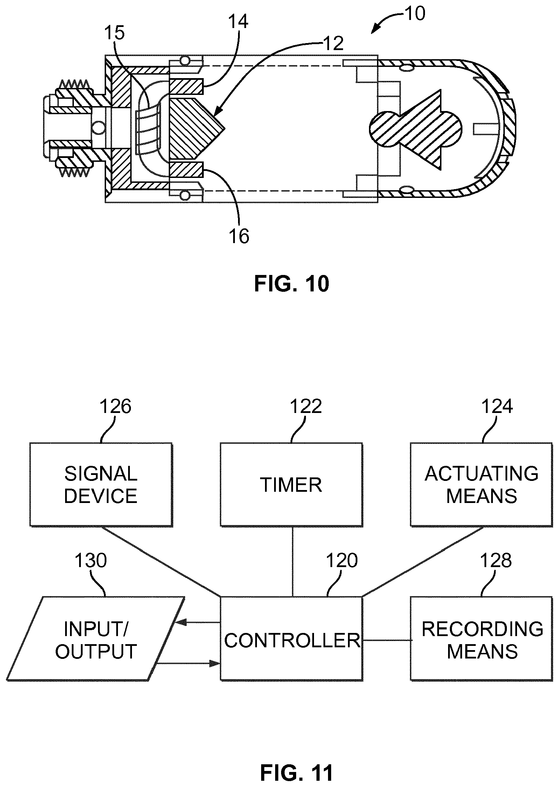

In FIG. 10, the dual-wick 14, 16 configuration of FIG. 9 allows for controlled saturation of the vaporization element, preventing both the overload or the drying out of the wick 14, 16 along the vaporization element 20. This not only prevents the burned or metallic taste from occurring, it also prevents the degradation of the wicks which may cause tank 17 failure.

As best illustrated in FIG, 10, the present embodiment of the tank 17 and reservoir 19 incorporates all the structures known by those skilled in the art and so will not be addressed here. Only the modifications to the prior art are described herein and it will be understood that such modifications may be applied to any cartridge/tank style reservoir, making then modifications substantially universal. FIG. 10 shows the embodiment of the bottom wall 21 has been modified to comprise a molded, convex or conical silicon bottom wall catch basin 12. Also, two wicks 14 and 16 extend along edges of the catch basin 12 at opposite positions along a circumference of the catch basin 12, each being diametrically opposite to the other. This configuration allows for the inhalant product in the tank 17 or reservoir 19 to be in contact with at least one of the dual wicks 14, 16 regardless of how much or how little product is in the reservoir 19 and regardless of what position the reservoir 19 is maintained. As stated above, the modifications prevent a burned taste from occurring and prevent degradation of the wicks 14, 16 which may cause failure of the tank 17 or reservoir 19.

As described above, the tank 17 provides a number of advantages, some of which have been described above and others of which are inherent in the invention.

In FIG. 11, the operational control of the assembly 10 can be accomplished with a controller 120 connected to a timer 122, actuating means 124, and signaling device 126. The controller 120 would ideally be either integral with or connected to a microprocessor 11 and would have a battery (e.g., nickel-cadmium, lithium, pulse modulated, etc. and/or combinations thereof). The timer 122, actuating means 124 and signaling device 126 could all be built-in or separate from the controller 120. In addition, a recording means 128 could be associated with or built-in the controller 120 so that a history of actuations and attempted non-prescribed actuations could be recorded for later analysis.

The controller 120 could be an electronic device, a programmable device, or other suitable device. In a preferred embodiment, the controller 120 could be programmable or pre-programmed with the dosage information and amounts for the active ingredient or drug to be delivered assembly 10. The number of actuations, length of vaporization, and optional control of time interval between actuations (e.g., one actuation every 3 minutes) into the controller 120 with input/output (I/O) device 130. The device 10 could be programmed to provide a dose each time the device is actuated. Optionally, the controller 120 can signal the actuator means 124 to lock-up and prevent actuation of the vaporization after the requisite number of actuations have occurred and during the periods when actuation is not supposed to occur. An actuation occurs when the vaporization device 10 is incited, put into action, or starts a process for vaporization of a vaporization substance. The process starts when a sensor senses a user's draw on the device, the sensing is sensed by, as an example, an airflow sensor or pressure sensor. During an active agent release or dose period, the controller 120 will sense at least one actuation of vaporization, and an actuation signaling the end of the vaporization process, and can, optionally, lock up and prevent actuation of the assembly 10 until a predetermined minimal time has passed.

Programming the proper dosage schedule is well within the level of ordinary skill in the art and would be analogous to products like programmable pacemakers and programmable implantable pumps. In addition, pre-set electronic devices are well understood in the art and are applicable in the practice of this invention where an electronic chip 132 is inserted in the controller 120 to control the active agent release/dosing/timing schedule. However, it should be understood that a major distinction between pacemakers and implantable pumps and vaporization devices and vaporization device assemblies is that the vaporization device or vaporization device assembly requires patient interaction with it. With pace makers and implantable pumps, the patient exercises no control over the treatment supplying unit. Therefore, the concept of dosing control of a vaporization device or vaporization device assembly as disclosed herein is a unique feature of this invention.

In addition, the electronic devices or chips could be preset with the active agent release/dosing schedule or could be settable or programmable with the dosing schedule by qualified medical personnel (e.g., Physician, Pharmacist, etc) or personnel at a company that supplies the vaporization devices or vaporization device assemblies.

In a particular embodiment of the invention, the controller 120 would accommodate at least one pluggable and/or replaceable pre-programmed chips or other circuitry 134. The at least one pluggable and/or replaceable pre-programmed chips or other circuitry 134 would supply the active agent release/dosing schedule for a particular vaporization substance in tank 17 and/or reservoir 19. Hence, different pluggable and/or replaceable chips 134 could be provided so that different timer/active agent release/doses for different drugs and different doses of a particular drug could be accommodated by the same assembly 10. The assembly 10 could have a new chip 134 plugged into the controller 120 with the new active agent/drug prescription or with a new prescription of the same active agent/drug at higher or lower doses. In this way, the assembly 10 could be reusable rather than disposable. In addition, the pluggable chip concept can benefit disposable inhalation device manufacture since the facility could make the same basic vaporization device or vaporization device assembly for a variety of active agents/drugs, and this device could be modified prior to packaging by plugging in a suitable chip 134 and filled tank 17.

The actuator means 124 could take a variety of forms. For example, the actuator means 124 could be temperature control device 136 that heats to a preset temperature and pens time intervals and cools after a preset number of actuations or present length of actuation, or a mechanical member that blocks actuation of vaporization. The timer 122 can monitor the time intervals between actuations so that the controller 120 can accurately control the actuator means 124.

The timer 122 will also monitor a time period for delivering the active agent/dose. In one embodiment, the timer 122 could cause the signaling device 126 to be activated at predetermined intervals dependent on the active agent release/dosing schedule. In another embodiment, the signaling device 126 would only be activated if assembly 10 is used within a preset time interval after the regularly scheduled active agent release/dosing time. In this way, the assembly 10 may not be subjected to an alarm if the active agent release/dosing schedule is complied with. In operation, if no use of the assembly 10 occurs during a preset time period allocated for active agent release/dosing, the controller 126 alerts the signaling device 126 to provide haptic feedback that the assembly is ready for use.

The haptic feedback provided by the signaling device 126 could be audible, visual, tactile, etc., or combinations thereof. The number and time of actuations could be recorded by recording means 129, such as a recorder 128. The recording means 129 could be a bubble memory, hysteresis memory, or other suit able memory device. The actuation information could then be downloaded to input/output unit 130 and printed off on a separate device (not shown) so the actuation history could be evaluated. The actuation information could also be downloaded onto a personal digital assistant (not shown), such as a mobile phone, or computer, or other equivalent. As will be explained below, the recording means 128 could also record information related to improperly attempted actuations (e.g., when attempts have been made to use the assembly 10 for more than the prescribed amount of active agent/drug by actuating the tank 17 additional times) and improperly performed actuations (e.g., when the assembly 10 is used without inspiring). In this way, the recorded history could aid in adjusting active ingredient/drug prescriptions to manage the care of a person dealing with a disease, illness, or other medical issue.

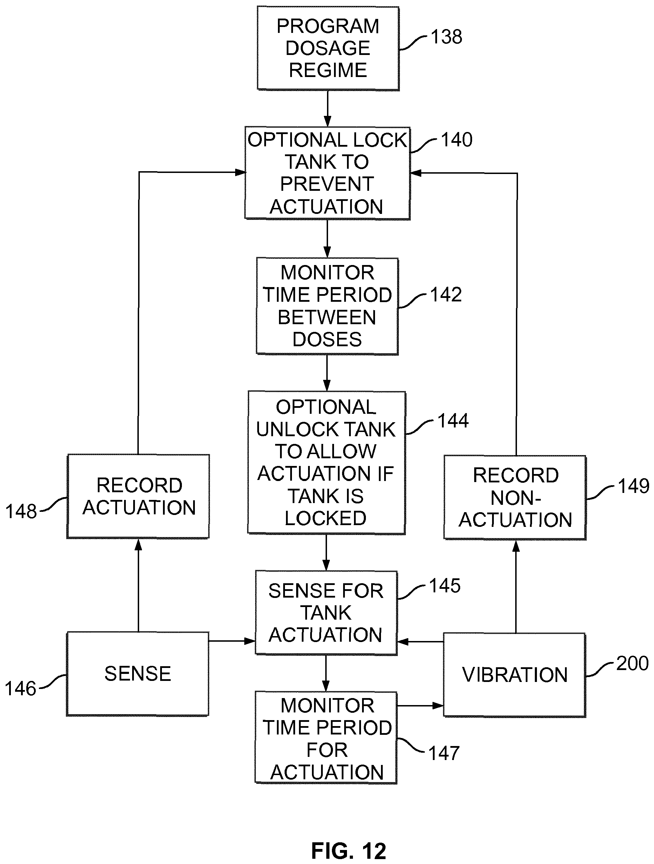

FIG. 12 shows the operation of the assembly 10 according to this disclosure. In step 138, the dosage regimen is programmed into the controller. The programmed dosage regimen varies according to the drug being delivered and how the assembly 10 is being used. Programming the controller could be performed at the manufacturer's facilities for many prescription drugs. Rather than computer programming, preset electronic components could be used as the "programmed" dosage regime 138. In optional step 140, the operation of the assembly 10 can be locked up until the preset dosing time. For example, the tank 17 of assembly 10 could be prevented from being actuated during the non-dose periods. In step 142, the time period between active agent release/doses is monitored. This is accomplished using a timer 122 and the preprogrammed time period for active agent release/dosing. In step 144, the assembly 10 is permitted to be actuated for a preset interval once the preprogrammed time period for active agent release/dosing arrives. This could be accomplished by allowing the temperature control device 136 to heat up heating element 137 and vaporize the vaporization substance in the tank 17 and/or reservoir 19. A temperature control device 139 is used to control the temperature of the heating element 137 to keep the flavor of the vaporized substance palatable. The temperature range of vaporization of cannabinoids and terpenes is far lower than that of the e-liquids that are used in standard electronic cigarette products, and to ensure consistent delivery of medicine this temperature must be properly controlled.

In step 145, actuation of the inhalation device is sensed and can be counted. A counting mechanism is disclosed in U.S. Patent No.5,020,527, and is hereby incorporated by reference. That counting mechanism could be used for the present disclosure. Sensing mechanisms are disclosed in U.S. Patent No. 5,676,129, which is hereby incorporated by reference. Specifically, as is shown in FIG. 1, the senor 27 could be a plurality of sensors, such as an optical sensor 146 that monitors the vaporization substance level in tank 17 and/or reservoir 19. When at least a portion of tank 17 (e.g., more than 0% of the tank contains the vaporization substance obstructs the optical sensor 146), the vaporization substance is vaporized by the heating element 137 being adapted to the microprocessor 11, the microprocessor 11 signaling the controller 120 to turn on the heating element 137, to vaporize the vaporization substance. The sensor is not limited to an optical sensor. The could also be a pressure sensor, air flow sensor, or any other type of sensor that starts or stops actuation of a device or process.

The heating element 137 heats to a temperature to change the physical phase of the vaporization substance without burning it. The heating element 137 is in electronic communication with the microprocessor 11 which is in electronic communication with the PCB 7. The temperature of the heating element 137 is controlled by electronic communications and can heat to the boiling point temperature of the vaporization substance. The temperature range can be between 200°F and 475°F, 210°F and 475°F, 215°F and 475°F, 220°F and 475°F, 225°F and 475°F, 200°F and 470°F, 200°F and 465°F, 200°F and 460°F, 200°F and 455°F, 200°F and 450°F, 200°F and 445°F, 225°F and 440°F, 225°F and 440°F, 225°F and 435°F, 225°F and 435°F, or any single temperature between any of the ranges above.

The time and number of actuations of the assembly 10 could be recorded at step 148. After the prescribed number of actuations occurs, operation of the assembly 10 can be, again, optionally, locked-up by returning to step 140 and no further actuations can be made until the prescribed interval between active agent release/doses passes.

The optional locking up the inhalation device can be achieved by any number of means. For example, the controller 120 could operate the heating element 137.

This would be especially useful where an airflow sensor 30 (not shown) senses an air stream passing through the airway/duct 50. By the microprocessor 11 shutting off the heating element 137 after the programmed or preset active agent/dose mount has been delivered through the airway/duct 50, the preset dose has been administered.

An additional and optional feature of this disclosure is to block circumventing a dosing schedule, as could occur if the assembly 10 was actuated during the time period the tank 17 is locked from actuation. A pressure sensor 28 can sense whether there is any vacuum pressure being exerted on the assembly 10 during actuation, as would be the case if the a draw or toke of air was pulled through the device. If no vacuum pressure is sensed coincident with the actuations, it is most likely that the active agent/dose was administered. The pressure sensor 28 would be electrically connected to the controller 120 so that the controller could direct the signaling vibration assembly 200 to provide haptic feedback. The event could also be recorded on recording means 129.

After the tank 17 is sensed for actuation, the time period from the time of actuation to the preset next active agent release/dose time can be monitored in step 147. Once the proper amount of the has elapsed, the vibration assembly 200 the assembly 10 can be turned on in step 148 to alert that the time period has elapsed, or alert that the assembly 10 has performed other operations based on the preset time and dose parameters.

In one embodiment, if the assembly 10 is used before the programmed or preset time has elapsed and the assembly 10 does not actuate, the non-actuation can be recorded in step 149.

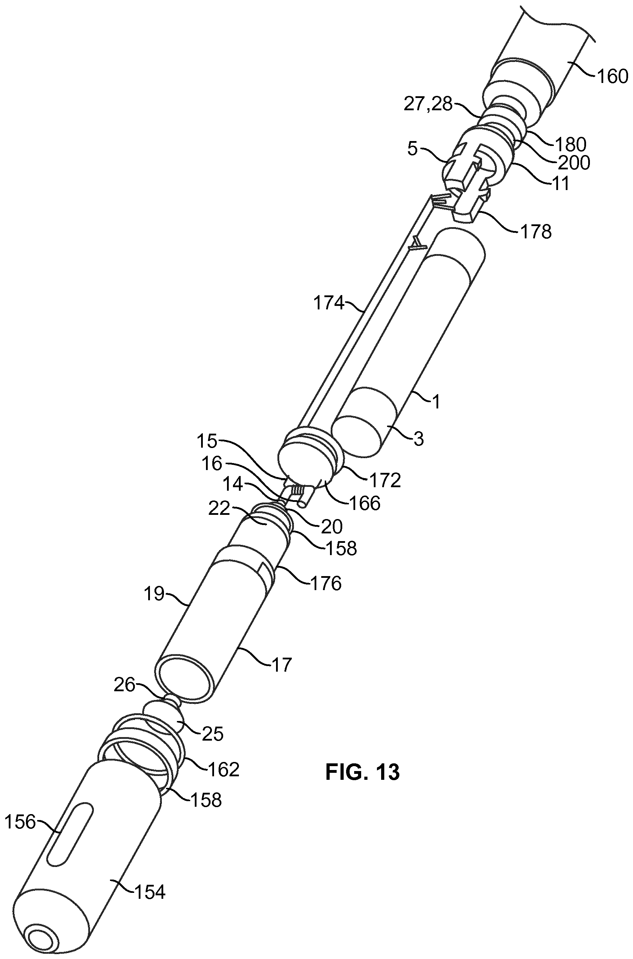

In FIG. 13, another embodiment of the disclosure is shown. The airflow sensor 30 (not shown) senses the airflow of air and/or vapor that contains the vaporization substance, and is electrically adapted to the microprocessor 11. The airflow sensor 30 aids in the precise control of the programmed or present active agent/drug dosage. The airflow sensor 30 comprises a first custom molded component 150 and a second molded component 152, each of which is made of a material that does not react with oxygen, such as silicon. The first custom molded component 150 and the second custom molded component 152 are spatially arranged in proximity to each other to control the draw pressure of the airflow through the assembly 10. The closer the first custom molded component 150 and the second custom molded component 152 are to each other, the harder the draw. The further apart the first custom molded component 150 and the second custom molded component 152 are, the easier the draw.

The airflow sensor senses the draw pressure, for instance, in kPa, to deliver the correct amount of vapor through the tip 29.

The controller 120 is adapted to the airflow sensor and can increase or decrease the draw pressure if the initial draw pressure sensed is above or below a kPA necessary to deliver an effective dose of the active agent. Sometimes a user is unable to create a draw pressure necessary to deliver an effective dose of the active agent because, for instance, the user's lungs do not function well. The controller 120 can be used to assist with the draw pressure required for effective dose delivery.

In one embodiment, at least one of the parts of assembly 10 is made of a medical grade material, such as plastic. The medical grade material is resistant to bacteria and other contamination.

Also in FIG. 13, assembly 10 comprises molded mouthpiece cap 154 that comprises a viewing window 156 to view the vaporization substance level in the a tank 17 and/ reservoir 19. Airflow through the assembly 10 is allowed through the tip 29. The molded mouthpiece cap 154 can be placed on the assembly 10 after the tank 17 and/or reservoir 19 is/are filled at a licensed facility.

A retaining ring 158, that may be made from a material that does not react with oxygen, such as silicon, is placed between the molded mouthpiece cap 154 and molded device body 160. The retaining ring 158 provides correct sealing for connection of molded mouthpiece cap 154 and molded device body 160. At least one molded reservoir plug 25, that can comprise a ball shaped end 26, ensures proper seating of retainer ring 158. The molded reservoir plug 25, that can be ball shaped 26, caps the tank 17 after the tank 17 is filled to prevent tank 17 of leaking or spilling. The molded reservoir plug can be pushed 6 mm into the tank 17 and the molded reservoir plug can function as a one-way valve to convert air pressure into a vacuum inside the tank 17.

Tank 17 can have a concave top molding design to improve accuracy when filling the reservoir 19. The concave molding design at top of reservoir 19 also reduces the ability of the vaporization substance to leak, during storage of assembly 10 or exposure of assembly 10 to high ambient temperatures, at the point where the molded reservoir 25, which can be ball shaped 26, caps the tank 17.

Retainer clip 164 is a molded component located at the bottom of the tank 17. At least one retainer clip 164 fastens a vaporization cup 166 to the tank 17 using a retainer clip assembly 168. The vaporization cup 166 may be made of an anti-static material, such as ceramic, and may be made of a medical grade material, comprises at least one vaporization substance flow control access port 170 that allow the vaporization substance to flow through the retainer clip assembly 168 and onto the vaporization element 20. The vaporization cup 166 may also act as a frame to support the vaporizing element 20, inside the assembly 10, which produces the flow of vapor to the mouthpiece 8.

In one embodiment, assembly 10 does not have a wick that is placed directly into reservoir 19. The retainer clip assembly 168 is a combination of components and comprises an aluminum retainer clip 182 and a screen 22 that interacts with the wicks 14, 16 in the vaporization device 10. The retainer clip assembly allows a controlled flow vaporization substance from the reservoir 19 or tank 17, through a reservoir flow control bracket 184, and onto the vaporization element 20. An advantage of the retainer clip assembly 168 is that it prolongs the useful life of the wicks 14, 16 because they never become over-saturated with vaporization substance.

The vaporization element 20 comprises wicks 14, 16 and a vaporization substance. The wicks 14, 16 can be made from a glass-filled fiber, or a cotton blend. The glass-filled fiber has a more consistent saturation rate and is less susceptible to burning from the coil 15, which can increase the life of the wick and palatability of the vaporization substance, particularly when the life of assembly 10 is nearing its end.

In one embodiment, coil 15 can be made from a nickel alloy or NiChrome wire. Coil 15 has a lower temperature of vaporization versus the NiChrome, which heats to 900 degrees Fahrenheit, which is far above the temperature of vaporization for most cannabinoids. This increased temperature can cause wasted heat and the burning of the active agent in the assembly.

This custom molded ceramic piece acts as the housing for the Vaporization Element (8) and retains any excess need state oil until vaporized on activation by the patient.

In one embodiment, the vaporization cup 166 also assists in heat dissipation and protection of the lifecycle of the vaporizing element 20. The coil 15 can heat to the programmed temperature and stops heating once the programmed time for heating is over and/or the programmed temperature is reached. The vaporization cup 166 can be made of a material that dissipates heat, such as ceramic. The material used to make vaporization cup 166 can assist with the cooling of the vaporization device 10 to ensure the vaporization substance is not burnt and does not continue to be vaporized after the vaporization time has commenced. The vaporization cup 166 comprises a cup retainer ring 172, that can be made of an anti-static material, such as ceramic. Cup retainer ring 172 provides a sealed barrier between the top half of the assembly 10, where the vaporization occurs) and the bottom half of the assembly 10, which contains the battery 1, microprocessor 11, and other electronic components.

In one embodiment, the power from batter 1 flows through a ribbon cable to the vaporizing element 20, the microprocessor 11, the vibration device 10, and the plurality of sensors 27.

In one embodiment, the ribbon cable 174 provides for a lower number of potential failure points that the individual wire design seen in standard disposable units on the market.

Assembly 10 can have a component assembly 176, to provide a frame that houses the microprocessor 11, the vibration device 10, and plurality of sensors 27.

The component assembly can also provide a frame clip 178 for the battery 1 to reduce vibration of the internal components of assembly 10 during transportation.

The vibration assembly 10 can be activated for 0.25-4 seconds, or any number of seconds within that range, i.e., 1.25, 1.5, 1.75, 2.0, 2.25, 2.5, 2.75, 3.0, 3.25, 3.5, 3.75, 4.0, or even smaller increments of time, following each three-second draw time.

In one embodiment, the plurality of sensors 27 are adapted to the PCB 7. The PCB 7 can comprise an LED indicator 180 and the pressure sensor 28. The controller 120 is electrically engaged with the LED indicator 180, and can turn it on at the start of assembly 10 activation (draw). The LED indicator 180 can remain on through the three-second activation, and blink to signify the end of the dose. The microprocessor 11 is electrically engaged with the LED indicator 180 and the vibrator 9. The microprocessor 11 can be programmed to turn on the LED indicator 180 before the vibrator 9 is turned on, after the vibrator 9 is turned on, or can turn on both the LED indicator 180 and vibrator 9 separately.

Pressure sensor 27 senses airflow and sends a signal to the microprocessor 11 the activation of assembly 10. Battery 11 provides power to the vaporization element 20 to turn on the LED indicator 180, and to begin the timer for the vibration device 10.

As to further manners of usage and operation of the present disclosure, the same should be apparent from the above description. Accordingly, no further discussion relating to the manner of usage and operation will be provided.

While an embodiment of the device, kit, assembly and method of use has been described in detail, it should be apparent that modifications and variations thereto are possible, all of which fall within the true spirit and scope of the invention. With respect to the above description then, it is to be realized that the optimum dimensional relationships for the parts of the invention, to include variations in size, materials, shape, form, function and manner of operation, assembly and use, are deemed readily apparent to one skilled in the art, and all equivalent relationships to those illustrated in the drawings and described in the specification are intended to be encompassed by the present disclosure.

Those skilled in the art will appreciate that the invention described herein is susceptible to variations and modifications other than those specifically described, and that each embodiment is also provided with features that may be applicable to other embodiments. It is to be understood that the invention includes all such variations and modifications that fall within its spirit and scope. The invention also includes all of the steps, features, compositions and compounds referred to or indicated in this specification, individually or collectively, and any and all combinations of any two or more of said steps or features.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

## Claims

1. A vaporization device comprising:
a vaporization substance delivery means comprising a battery, a coil, a vaporization chamber, a plurality of wicks, and a tank having a reservoir configured for housing a vaporization substance, where the vaporization substance delivery means vaporizes the vaporization substance;
the vaporization substance delivery means disposed in electronic communication with a microprocessor that controls actuation of the vaporization substance delivery means based on a prescribed dosage regimen for the vaporization substance;
wherein the prescribed dosage regimen comprises
a preset dose delivery defined by a time duration of an actuation, and
a time delay before initiation of a subsequent preset dose delivery;
a timer in electronic communication with said microprocessor for tracking the time delay;
a recording means in electronic communication with said microprocessor for recording the number of actuations and the time duration of each actuation;
wherein the microprocessor prevents actuation of said vaporization substance delivery means after an actuation has been completed until the time delay has passed;
means for the microprocessor to verify the preset dose delivery has been completed;
a vibrator in electronic communication with said microprocessor for actuating said vibrator; and
a vibration control assembly.

2. A vaporization substance delivery device comprising:
at least one battery with a mouthpiece end and an opposing vibration end, a first spacer at the mouthpiece end and a second spacer at the vibration end;
at least one microprocessor programmed to deliver a preset dosage of an active agent;
at least one printed circuit board, at least one vibrator and the at least one microprocessor housed in the second spacer;
a coil holder configured to accommodate a ceramic coil, a vaporization chamber, and a plurality of wicks;
a ceramic coil for vaporizing substances accommodated by the coil holder;
a tank having a reservoir, a first and a second end, wherein the reservoir is configured for housing vaporization substances;
a reservoir plug which is accommodated in a first end of the tank; and
a pressure sensor in pneumatic communication with the reservoir of the tank, wherein the at least one battery at the vibration end is adjacent to and in electronic communication with the printed circuit board, the vibrator and the microprocessor which are contained in the second spacer,
wherein the battery at the mouthpiece end is in electronic communication with the ceramic coil, and wherein the battery at the mouthpiece end is contiguous with the first spacer which separates the battery from the tank and the mouthpiece.

3. The vaporization device of claim 1, wherein the microprocessor is programmed to signal that the preset dose amount has been delivered by the vaporization substance delivery means by actuating said vibrator.

4. The vaporization device of claim 2, wherein the microprocessor is programmed to activate the vibrator in response to receiving a signal indicating an operation selected from the group of operations consisting of at the initiation of inhalation, at the initiation of inhalation and through the end of a draw, and at the end of a draw.

5. The vaporization device of claim 4, wherein the microprocessor is programmed to activate the vibrator at the end of the draw and disconnects the battery from the ceramic coil thus preventing further vaporization of the substances.

6. The vaporization device of claim 5, wherein the microprocessor is programmed to activate the vibrator based on receipt of a preprogrammed pressure signal or the time elapsed after initiation of inhalation.

7. The vaporization device of claim 6, wherein the device comprises medical grade materials.

8. The vaporization device of claim 7, wherein the medical grade materials comprise materials that are bacteria resistant.

9. The vaporization device of claim 1, further comprising a screen, wherein the vaporization substance temporarily adheres to the screen though surface tension.

10. The vaporization device of claim 9, wherein the screen is a capillary screen.

11. The vaporization device of claim 10, wherein the screen is made of an anti-static material.

12. The vaporization device of claim 10, wherein the screen is made of medical grade materials.

13. The vaporization device of claim 10, wherein the screen is made of ceramic.

14. The vaporization device of claim 1, wherein the time duration of an actuation is at least three seconds.

15. The vaporization device of claim 1, wherein the time duration of an actuation is for at most three seconds.

16. The vaporization device of claim 1, wherein the microprocessor is further programmed to control temperature of vaporization.

17. The vaporization device of claim 16, wherein the temperature of vaporization does not exceed 475°F.

18. The vaporization device of claim 16, wherein the microprocessor limits the temperature of vaporization below a temperature that overheats or bums the vaporization substance.

19. The vaporization device of claim 1, wherein the vibration control assembly comprises: a spacer, a printed circuit board, a vibrator, and a microprocessor for use in controlling the time at which the vibrator vibrates, the duration of the vibration, the strength of the vibration, and the pattern of the vibration, wherein the spacer is formed of a dampening material selected from the group of dampening materials consisting of plastic, rubber, silicon, and mixes thereof, and wherein the spacer houses the printed circuit board, the vibrator and the microprocessor.

20. The vaporization device of claim 1, wherein the battery maintains a voltage range to actuate consistent preset dose delivery amounts for 200 doses.

21. The vaporization device of claim 1, wherein the battery is rated at 260mAh.

22. The vaporization device of claim 1, wherein the battery is a lithium ion battery.

23. The vaporization device of claim 1, wherein the vaporization substance delivery means further comprises an airflow control assembly.

24. The vaporization device of claim 23, wherein the airflow control assembly comprises a first molded silicon component and a second molded silicon component that are spatially arranged to regulate draw pressure.

25. The vaporization device of claim 23, wherein the airflow control assembly controls airflow through the vaporization device to deliver the preset dose delivery.

26. The vaporization device of claim 23, wherein the airflow control assembly increases airflow through the vaporization device to deliver the preset dose delivery.

27. The vaporization device of claim 23, wherein the airflow control assembly decreases airflow through the vaporization device to deliver the preset dose delivery.

28. The vaporization device of claim 9, wherein the screen contacts the plurality of wicks.

29. The vaporization device of claim 9, wherein the screen delivers the vaporization substance to the plurality of wicks.

30. The vaporization device of claim 9, further comprising a screen retainer clip assembly.

31. A method for controlling vibration within the vaporization device of claim 1, the method comprising the steps of:
receiving, at a microprocessor, a signal from a pressure sensor in pneumatic communication with a reservoir of the vaporization device;
determining, at the microprocessor, at least one of an initiation of inhalation and an end of a draw of the vaporization device based on the signal from the pressure sensor; and
activating a vibrator based on the at least one of the initiation of inhalation and the end of a draw of the vaporization device.

32. The method of claim 31, wherein the activating step further comprises electrically connecting the battery to a coil to enable vaporization.

33. The method of claim 31, further comprising the step of deactivating the vibrator based on the at least one of the initiation of inhalation and the end of a draw of the vaporization device.

34. The method of claim 33, wherein the deactivating step further comprises disconnecting a battery from a coil to prevent vaporization.

35. The method of claim 31, further comprising the step of receiving, at the microprocessor, a second signal related to a duration of time.

36. The method of claim 35, further comprising the step of determining, at the microprocessor, at least one of an initiation of inhalation and an end of a draw of the vaporization device based on the signal from the pressure sensor and the second signal related to a duration of time.

37. A vaporization device assembly comprising:
a battery with a mouthpiece end and an opposing vibration end, a first dampening spacer at the mouthpiece end and a second dampening spacer at the vibration end;
a printed circuit board, a vibrator and a microprocessor housed in the second dampening spacer;
said microprocessor, in electronic communication with said a vaporization substance delivery means;
the vaporization substance delivery means comprising the battery, a ceramic coil, a vaporization chamber, a plurality of wicks, and a tank having a reservoir configured for housing a vaporization substance, where the vaporization substance delivery means is programmed or preset with dose amount and time of dose information for the vaporization substance, said dose and time of dose information setting forth a number of actuations of said vaporization substance delivery means to be provided at prescribed intervals;
a timer, in electronic communication with said printed circuit board and said microprocessor, for tracking the time between actuations of said vaporization substance delivery means;
a coil holder configured to accommodate the ceramic coil, the vaporization chamber, and the plurality of wicks;
the ceramic coil for vaporizing substances accommodated by the coil holder;
the tank having a first and a second end;
a reservoir plug which is accommodated in the first end of the tank; and
a sensor for detecting at least one operation selected from the group of operations consisting of the beginning of inhalation by a user, the end of inhalation by a user, the beginning of vaporization and the end of vaporization, wherein the battery at the vibration end is adjacent to and in electronic communication with the printed circuit board, the vibrator and the microprocessor which are contained in the second dampening spacer, wherein the battery at the mouthpiece end is in electronic communication with the ceramic coil, and wherein the battery at the mouthpiece end is contiguous with the first dampening spacer which separates the battery from the tank and the mouthpiece.

38. The vaporization device of claim 37, wherein the sensor is selected from the group of sensors consisting of capacitive, photoelectric, and fiber optic.

39. A kit comprising:
the vaporization device of claim 1;
a charging case comprising a lid, a cradle and a base for charging the electronic cigarette; and
a plurality of tanks filled with vaporizable substance.
